(19) Europäisches Patentamt
European Patent Office
Office européen des brevets

(11) Numéro de publication : **0 023 454**
**B1**

(12) # FASCICULE DE BREVET EUROPÉEN

(45) Date de publication du fascicule du brevet : **22.06.83**

(51) Int. Cl.$^3$ : **C 07 D307/93**, C 07 D307/60// C07C149/31

(21) Numéro de dépôt : **80401093.2**

(22) Date de dépôt : **23.07.80**

(54) **Procédé de préparation de la 6,6-diméthyl-4-hydroxy-3-oxabicyclo (3.1.0)hexan-2-one et de ses éthers sous toutes leurs formes stéréoisomères.**

(30) Priorité : **31.07.79 FR 7919654**

(43) Date de publication de la demande :
**04.02.81 Bulletin 81/05**

(45) Mention de la délivrance du brevet :
**22.06.83 Bulletin 83/25**

(84) Etats contractants désignés :
**AT BE CH DE GB IT LI NL SE**

(56) Documents cités :
**EP A 0 004 493**
**BE A 873 106**
**BE A 873 201**
**BE A 873 798**
**JOURNAL OF ORGANIC CHEMISTRY, vol. 43, no. 22, 27 octobre 1978, pages 4323-4328 Washington, U.S.A. P.R. ORTIZ DE MONTELLANO et al. : « Base-catalyzed isomerization of cis- and trans-2,2-dimethyl-3-formylcyclopropanecarboxylates. Nature of the base-stable cis intermediate »**
**JOURNAL OF THE AMERICAN CHEMICAL SOCIETY, vol. 96, no. 17, 21 août 1974, pages 5627, 5628 Washington, U.S.A. T. COHEN et al. : « A laboratory model for the biosynthesis of cyclopropane rings. Copper catalyzed cyclopropanation of olefins by sulfur ylides »**

**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, rue Jean Moulin**
**F-94300 Vincennes (FR)**
Inventeur : **Demoute, Jean-Pierre**
**249 bis rue de Rosny**
**F-93100 Montreuil s/Bois (FR)**

(74) Mandataire : **Niel, Michel et al**
**ROUSSEL-UCLAF boîte postale no 9 102, route de Noisy**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 023 454 B1

Procédé de préparation de la 6,6-diméthyl-4-hydroxy-3-oxabicyclo (3.1.0) hexan-2-one et de ses éthers sous toutes leurs formes stéréoisomères

La présente invention concerne un procédé de préparation de (1,5) 6,6-diméthyl 4-hydroxy 3-oxa bicyclo [3.1.0] hexan-2-one et de ses éthers sous toutes leurs formes stéréoisomères.

L'invention a pour objet un procédé de préparation de composés de configuration (1RS, 4RS, 5SR), (1R, 4R, 5S) ou (1S, 4S, 5R), de formule I

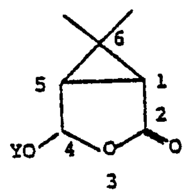

(I)

dans laquelle Y représente un atome d'hydrogène ou le reste organique Z d'un alcool ZOH éventuellement chiral, caractérisé en ce que l'on fait réagir, en présence d'un agent acide, la 5RS-hydroxy 2,5-dihydrofuran-2-one, de formule II

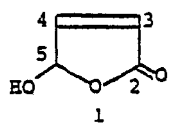

(II)

— soit avec un alcool achiral ZOH, soumet l'éther résultant, en milieu anhydre, à l'action d'un isopropylidène sulfurane de formule III

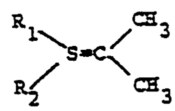

(III)

dans laquelle $R_1$ et $R_2$, identiques ou différents, représentent soit des groupements aromatiques monocycliques éventuellement substitués et notamment des radicaux phényles éventuellement substitués par des méthyles, soit des groupements isopropyles, soit des groupements alcoyles tertiaires, pour obtenir le composé (I) de structure (1RS, 4RS, 5SR) dans lequel Y représente Z, puis hydrolyse, si désiré, ce composé en milieu acide pour obtenir le composé (I) correspondant dans lequel Y représente de l'hydrogène,

— soit avec un alcool chiral optiquement actif ZOH, pour obtenir un mélange d'éthers stéréoisomères dus à l'existence du carbone asymétrique en position 5, mélange qui peut être plus riche en l'un des deux diastéréoisomères, sépare par un moyen physique les éthers diastéréoisomères formés, les fait réagir en milieu anhydre avec un isopropylidène sulfurane de formule III pour obtenir les composés de formule I correspondants de configuration (1R, 4R, 5S) ou (1S, 4S, 5R), formule I dans laquelle Y représente Z que l'on hydrolyse, si désiré, en milieu acide, pour obtenir les composés (I) correspondants, dans lesquels Y représente de l'hydrogène.

L'agent acide en présence duquel on fait réagir la 5RS-hydroxy 2,5-dihydro furan-2-one et l'alcool ZOH est choisi avantageusement dans le groupe constitué par les acides sulfoniques, l'acide sulfurique, l'acide chlorhydrique et l'acide phosphorique.

Dans un des modes d'exécution préféré de l'invention, l'agent acide utilisé est l'acide paratoluène sulfonique.

Comme isopropylidène sulfurane on utilise avantageusement le diphényl isopropylidène sulfurane.

Les isopropylidènes sulfuranes utilisés dans le procédé de l'invention sont préparés par action d'une base sur le sel de sulfonium correspondant, selon les procédés connus. Comme sel de sulfonium, on utilise avantageusement un fluoroborate. Un exemple d'une telle préparation est donné, plus loin, dans la partie expérimentale, à titre indicatif.

La réaction de la furanone et du sulfurane est effectuée, de préférence, au sein d'un solvant choisi dans le groupe constitué par l'éther diéthylique ou diméthylique du diéthylèneglycol, l'éther éthylique, le diméthylsulfoxyde, le tétrahydrofurane, le diméthoxyéthane. L'utilisation du tétrahydrofurane est particulièrement avantageuse.

Cette réaction aboutissant à la formation du cycle cyclopropanique est effectuée avantageusement à une température comprise entre − 90 °C et − 30 °C.

L'hydrolyse des éthers de formule (I) est effectuée avantageusement à l'aide d'un acide fort, en présence d'eau et au sein d'un solvant miscible à l'eau et capable de dissoudre le composé à hydrolyser.

L'acide fort utilisé pour hydrolyser les éthers de formule (I) est choisi notamment dans le groupe

constitué par les acides sulfoniques, l'acide sulfurique, l'acide chlorhydrique et l'acide phosphorique.

Le solvant au sein duquel est effectuée l'hydrolyse des éthers de formule (I) est choisi notamment dans le groupe constitué par les alcanols, le dioxane, le tétrahydrofurane, le diméthylformamide et certaines cétones aliphatiques.

L'invention a également pour objet un procédé tel que défini précédemment, caractérisé en ce que le reste d'alcool achiral Z est un reste méthyle et que l'on fait réagir alors la 5(RS)-méthoxy 2,5-dihydrofuran-2-one avec le diphényl isopropylidène sulfurane, pour obtenir le (1RS, 5SR) 6,6-diméthyl 4(RS) méthoxy 3-oxa bicyclo [3.1.0] hexan-2-one, que l'on soumet à une hydrolyse acide pour obtenir le (1RS, 5SR) 6,6-diméthyl 4(RS) hydroxy 3-oxa bicyclo [3.1.0] hexan-2-one.

L'invention a aussi pour objet un procédé tel que décrit précédemment, caractérisé en ce que le reste d'alcool chiral Z est un reste d'alcool S (α-méthyl 3-phénoxyphényl) méthylique, en ce que l'on fait alors réagir cet alcool avec la 5RS-hydroxy 2,5-dihydrofuran-2-one pour obtenir un mélange de 5R-[1S(3-phénoxyphényl) α-méthyl méthoxy] 2,5-dihydrofuran-2-one et de 5S-[1S (3-phénoxyphényl) α-méthyl méthoxy] 2,5-dihydrofuran-2-one plus riche en isomère « 5R » qu'en isomère « 5S », en ce que l'on sépare par chromatographie l'isomère 5R-[1S (3-phénoxyphényl) α-méthyl méthoxy] 2,5-dihydrofuran-2-one, en ce que l'on fait réagir ce composé avec le diphényl isopropylidène sulfurane, pour obtenir le (1R, 5S) 6,6-diméthyl 4R-[1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1R, 5S) 6,6-diméthyl 4R-hydroxy 3-oxa bicyclo [3.1.0] hexan-2-one.

L'invention a aussi pour objet un procédé analogue au procédé décrit ci-dessus mais caractérisé en ce que l'on sépare par chromatographie l'isomère 5S-[1S(3-phénoxyphényl) α-méthyl méthoxy] 2,5-dihydrofuran-2-one pour obtenir, après réaction avec le diphényl isopropylidène sulfurane le (1S, 5R) 6,6-diméthyl 4S-[1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1S, 5R) 6,6-diméthyl 4(S)-hydroxy 3-oxa bicyclo [3.1.0] hexan-2-one.

L'invention a également pour objet un procédé tel que décrit précédemment, caractérisé en ce que, le reste d'alcool chiral Z est un reste l-menthyle, en ce que l'on fait réagir cet alcool avec la 5RS-hydroxy 2,5-dihydrofuran-2-one, obtient un mélange de 5R [1R, 2S, 5R] 2-prop-2-yl 5-méthyl cyclohexyloxy 2,5-dihydrofuran-2-one et de 5S [1R, 2S, 5R] 2-prop-2-yl 5-méthyl cyclohexyloxy 2,5-dihydrofuran-2-one, plus riche en isomère 5R qu'en isomère 5S, en ce que l'on sépare par cristallisation l'isomère 5R [1R, 2S, 5R] 2-prop-2-yl 5-méthyl cyclohexyloxy 2,5-dihydrofuran-2-one, en ce que l'on fait réagir ce composé avec le diphényl isopropylidène sulfurane pour obtenir le (1R, 4R, 5S) 6,6-diméthyl-4[(1R, 2S, 5R) 2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1R, 5S) 6,6-diméthyl 4R-hydroxy 3-oxa bicyclo [3.1.0] hexan-2-one.

L'invention a aussi pour objet un procédé tel que décrit ci-dessus, mais caractérisé en ce que, après avoir séparé par cristallisation l'isomère R, on sépare par chromatographie le 5S-[1R, 2S, 5R] 2-prop-2-yl 5-méthyl cyclohexyloxy 2,5-dihydrofuran-2-one pour obtenir après réaction avec le diphényl isopropylidène sulfurane le (1S, 4S, 5R) 6,6-diméthyl 4-[(1R,2S,5R) 2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1S, 5R) 6,6-diméthyl 4S-hydroxy 3-oxa bicyclo [3.1.0] hexan-2-one.

Dans le procédé selon l'invention, des méthodes analytiques telles que la chromatographie en phase liquide à haute performance (ou H.P.L.C.) permettent de se rendre compte que la réaction d'un alcool chiral optiquement actif avec la 5RS-hydroxy 2,5-dihydro furan-2-one donne lieu à une induction asymétrique.

Le produit brut de réaction est ainsi plus riche en l'un des deux diastéréoisomères. Par exemple, dans le cas des éthers d'alcool S α-méthyl 3-phénoxyphényl méthylique, on constate que le rapport :

$$\frac{\text{Forme R}}{\text{Forme R + Forme S}} = 67\,\%\ \text{au lieu de}\ 50\,\%\ \text{théorique}$$

et que dans le cas des éthers du l-menthol, ce même rapport

$$\frac{\text{Forme R}}{\text{Forme R + Forme S}} = 59\,\%.$$

L'existence de ces deux cas d'induction asymétrique montre que le choix d'un alcool chiral convenable permet d'obtenir un mélange plus riche dans la forme stéréochimique désirée et facilite ainsi l'isolement de cette forme.

Il est à noter, dans le cas de l'éther de l-menthyle, qu'après séparation du 5R-[1R, 2S, 5R] 2-prop-2-yl 5-méthyl cyclohexyloxy 2,5-dihydrofuran-2-one, les liqueurs mères résultantes, après chauffage en milieu acide, conduisent à nouveau à un mélange d'isomère R et d'isomère S dans une proportion voisine de celle du mélange initial et que l'on peut ainsi isoler à nouveau l'isomère R et obtenir en plusieurs jets successifs un rendement élevé en cet isomère R.

Ce phénomène est d'ordre général lorsque par une méthode physique telle que cristallisation ou chromatographie on sépare une des deux formes stéréoisomères, on peut ensuite, par chauffage des

liqueurs mères en milieu acide, atteindre à nouveau l'équilibre initial entre forme R et S puis isoler la forme stéréoisomère initialement séparée. La répétition de ce processus permet d'obtenir un rendement élevé en l'une des formes stéréoisomères.

Par ailleurs, la stéréosélectivité de la réaction du sulfurane sur les composés II est à signaler particulièrement car elle permet d'obtenir les composés (I) sous la seule forme stéréoisomère désirée, sans aucune réaction secondaire.

Sans que cela ne délimite en rien le domaine de l'invention, on peut dire que la stéréosélectivité de la réaction est liée à ce que l'attaque primaire du sulfurane sur le carbone en position 4 de la furanone (II) a lieu uniquement sous forme trans par rapport au groupement OZ en position 5. La cyclisation ultérieure conduit à un dérivé bicyclo-hexyl-[3.1.0] de structure cis, dans lequel le groupement Oz est exo. Finalement, bien que le composé (I) comporte trois centres d'asymétrie, le procédé de l'invention permet de n'obtenir qu'un racémique, lorsque l'alcool ZOH utilisé est achiral, ou un des deux énantiomères correspondants, lorsque l'alcool ZOH utilisé est chiral et que l'on a séparé au préalable les deux éthers diastéréoisomères issus de la 5-hydroxy 2,5-dihydro furan-2-one (II).

Ainsi le procédé de l'invention présente le grand avantage, au départ de réactifs simples et peu coûteux et en deux étapes réactionnelles seulement, d'ouvrir un accès aux formes stéréoisomères désirées, des éthers de (1,5) 6,6-diméthyl 4-hydroxy 3-oxa bicyclo [3.1.0] hexan-2-one, et par hydrolyse, aux formes stéréoisomères des dérivés hydroxylés correspondants.

Ces différents composés de formule I trouvent notamment leur utilité dans la préparation des produits de formule I correspondants, dans laquelle Y est hydrogène, produits connus (voir par exemple, le brevet français n° 1 580 474) et permettant la préparation d'acides cyclopropane carboxyliques diversement substitués, acides qui par estérification conduisent à des produits insecticides très actifs.

Les exemples suivants illustrent l'invention sans la limiter.

Exemple 1 : (1RS, 5SR) 6,6-diméthyl 4-(RS) méthoxy 3-oxa bicyclo [3.1.0] hexan-2-one

Stade A : 5(RS) méthoxy 2,5-dihydro furan-2-one

Ce composé peut être obtenu selon la méthode décrite par H. S. Schroeter et col. Liebigs Ann. Chem., *697*, 42 (1966).

Stade B : (1RS, 5SR) 6,6-diméthyl 4-(RS) méthoxy 3-oxa bicyclo [3.1.0] hexan-2-one.

On agite, sous atmosphère inerte, 350 mg de 5-(RS) méthoxy 2,5-dihydro furan-2-one dans 15 $cm^3$ de tétrahydrofurane, refroidit à − 90 °C, introduit lentement la solution de diphényl isopropylidène sulfurane maintenue à − 70 °C et préparée comme décrit ci-après, laisse une heure à − 78 °C et une heure à − 50 °C, puis verse sur une solution aqueuse de phosphate monosodique, extrait au chlorure de méthylène, concentre à sec la phase organique sous pression réduite, chromatographie le résidu sur silice en éluant par un mélange éther de pétrole-éther éthylique (1-1), recueille 480 mg de diphényl sulfure, 260 mg de (1RS, 5SR) 6,6-diméthyl 4-(RS) méthoxy 3-oxa bicyclo [3.1.0] hexan-2-one attendu et 100 mg de lactone n'ayant pas réagi.

Le diphényl isopropylidène sulfurane utilisé au départ de l'exemple 1 a été préparé comme suit :

On agite, sous atmosphère inerte, 950 mg de tétrafluoroborate d'isopropyl diphényl sulfonium dans 15 $cm^3$ de tétrahydrofurane, refroidit à − 70 °C et introduit, goutte-à-goutte, 2 $cm^3$ de solution de terbutyl-lithium à 1,6 M dans le pentane, laisse 30 minutes sous agitation à − 70 °C, pour compléter la réaction.

Exemple 2 : (1R, 5S) 6,6-diméthyl 4-(R) [1S (3-phénoxyphényl) α-méthyl méthoxy] 3-oxa bicyclo [3.1.0] hexan-2-one

Stade A : 5-R [1S (3-phénoxyphényl) α-méthyl méthoxy] 2,5-dihydrofuran-2-one.

On agite 6,5 g d'alcool S α-méthyl (3-phénoxyphényl) méthylique, 3,5 g de 5-(RS) hydroxy-2-(5H) furane, 180 mg d'acide paratoluène sulfonique et 100 $cm^3$ de benzène anhydre, chauffe au reflux pendant trois heures, refroidit, neutralise avec de la triéthylamine, concentre à sec sous pression réduite, obtient 10 g d'huile que l'on chromatographie sur silice en éluant par un mélange de benzène et d'acétate d'éthyle (95-5) et isole 5,3 g de 5-R [1S (3-phénoxyphényl) α-méthyl méthoxy] 2,5-dihydrofuran-2-one puis 2 g de 5S [1S (3-phénoxyphényl) α-méthyl méthoxy] 2,5-dihydro furan-2-one.

Caractéristiques de l'isomère 5-R

Spectre de R.M.N. ($CDCl_3$).

Pics à 6,07-6,17 p.p.m. attribués à l'hydrogène en 3 de l'hétérocycle.

Pics à 6,84-7,5 p.p.m. attribués à l'hydrogène en 4 de l'hétérocycle.

Pic à 5,72 p.p.m. attribué à l'hydrogène en 5 de l'hétérocycle.
Pics à 1,47-1,57 p.p.m. attribués aux hydrogènes du méthyle.
Pics à 4,77-4,87-4,97-5,07 p.p.m. attribués à l'hydrogène benzylique.
Pics de 6,83 à 7,5 p.p.m. attribués aux hydrogènes des noyaux aromatiques.

Caractéristiques de l'isomère 5-S

Spectre de R.M.N. ($CDCl_3$).

Pics à 1,41-1,48 p.p.m. attribués aux hydrogènes du méthyle.
Pics à 4,66-4,76 ; 4,87-4,96 p.p.m. attribués à l'hydrogène benzylique.
Pic à 6,08 p.p.m. attribué à l'hydrogène en position 2 de la furanone.
Pics à 6,06-6,16 p.p.m. attribués à l'hydrogène en position 4 de la furanone.
Pics à 6,75-7,4 p.p.m. attribués à l'hydrogène en position 3 de la furanone.
Pics de 6,75 à 7,4 p.p.m. attribués aux hydrogènes des noyaux aromatiques.

Stade B : (1R, 5S) 6,6-diméthyl 4-(R) [1S-(3-phénoxyphényl) α-méthyl méthoxy] 3-oxa bicyclo [3.1.0] hexan-2-one

On agite, sous atmosphère inerte, 400 mg de 5-R [1S (3-phénoxyphényl) α-méthyl méthoxy] 2,5-dihydrofuran-2-one dans 15 $cm^3$ de tétrahydrofurane, refroidit à − 70 °C, introduit lentement une solution ≃ 2,07 M de diphényl isopropylidène sulfurane préparée comme à l'exemple 1 mais à partir de 655 mg de sel de sulfonium et 1,5 $cm^3$ de terbutyl-lithium. Après une heure à ≃ 70 °C, on verse le mélange réactionnel sur une solution aqueuse de phosphate monosodique, extrait au chlorure de méthylène, lave, sèche, concentre à sec, cristallise le résidu dans un mélange éther de pétrole-éther éthylique (7-3) et recueille 310 mg de (1R, 5S) 6,6-diméthyl 4-(R) [1S (3-phénoxyphényl) α-méthyl méthoxy] 3-oxa bicyclo [3.1.0] hexan-2-one. F = 110 °C.

En opérant de façon analogue à celle de l'exemple 2, au départ du 5-S [1S (3-phénoxyphényl) α-méthyl méthoxy] 2,5-dihydrofuran-2-one après purification par chromatographie sur gel de silice, en éluant avec un mélange d'éther de pétrole (eb. = 35-70 °C) et d'éther éthylique (7-3) on obtient le (1S, 5R). 6,6-diméthyl 4-(S) [1S (3-phénoxyphényl) α-méthyl méthoxy] 3-oxa bicyclo [3.1.0] hexan-2-one.

Exemple 3 : (1R, 5S) 6,6-diméthyl 4-R-hydroxy 3-oxa bicyclo [3.1.0] hexan-2-one

On agite à 20/25 °C, pendant deux heures, 2 g du produit (1R, 5S) de l'exemple 2, dans 20 $cm^3$ d'acétone et 5 $cm^3$ d'acide chlorhydrique aqueux N, refroidit à 0, + 5 °C, alcalinise à pH ≃ 8 avec du bicarbonate de sodium, lave avec du benzène pour éliminer l'alcool α-méthyl 3-phénoxy benzylique, acidifie la phase aqueuse à pH 1,5-2 avec de l'acide chlorhydrique concentré, extrait à l'acétate d'éthyle, lave la phase organique à l'eau, la sèche, la concentre à sec sous pression réduite et obtient 800 mg de (1R, 5S) 6,6-diméthyl 4-R-hydroxy 3-oxa bicyclo [3.1.0] hexan-2-one, F = 120 °C · $[\alpha]_D$ = − 110° (c = 1 %, diméthylformamide).

Exemple 4 : (1R, 4R, 5S) 6,6-diméthyl 4-[(1R, 2S, 5R) 2-prop-2-yl 5-méthyl cyclohexyloxy] 3-oxa bicyclo [3.1.0] hexan-2-one

Stade A : 5R[(1R, 2S, 5R) 2-prop-2-yl 5-méthyl cyclohexyloxy] 2,5-dihydrofuran-2-one.

On chauffe au reflux 32,5 g de l-menthol, 21 g de 5-hydroxy 2,5-dihydro furan-2-one, 0,2 g d'acide paratoluène sulfonique et 300 $cm^3$ de benzène, de manière à éliminer par entraînement azéotropique l'eau formée. On ajoute 4 g de 5-hydroxy 2,5-dihydro furan-2-one, maintient au reflux encore pendant une heure, refroidit, lave la solution organique au bicarbonate de sodium, puis à l'eau, sèche, concentre à sec sous pression réduite, dilue le résidu obtenu (51,6 g) dans 100 $cm^3$ d'éther de pétrole (eb. = 35-70 °C), concentre par distillation, laisse pendant 17 heures à 0 °C, isole par essorage les cristaux formés et obtient 15 g de produit attendu (F = 76 °C).

On concentre les liqueurs mères à sec, dilue le résidu dans 150 $cm^3$ de benzène, ajoute 200 mg d'acide paratoluène sulfonique, porte le mélange réactionnel au reflux en éliminant l'eau formée, refroidit, lave au bicarbonate, à l'eau, sèche, concentre à sec, cristallise le résidu dans l'éther de pétrole (eb. = 35-70 °C) et obtient 10,35 g de produit attendu F = 76 °C.

On fait subir aux liqueurs mères un traitement analogue à celui indiqué ci-dessus et obtient 5,7 g de produit attendu F = 76 °C.

Un dernier traitement analogue fournit 3,4 g de produit attendu F = 76 °C.

En résumé, on a obtenu 34,45 g de produit cristallisé pur F = 76 °C.

Le 5R [(1R, 2S, 5R) 2-prop-2-yl 5-méthyl cyclohexyloxy] (5H) furan-2-one ainsi obtenu possède les caractéristiques suivantes :
$[\alpha]_D$ = − 139° (c = 1,5 %, chloroforme).

Spectre de R.M.N. (deutérochloroforme).

Pics à 0,75-0,86 p.p.m. ; 0,86-1,0 p.p.m. ; 0,83-0,94 p.p.m. attribués aux hydrogènes des méthyles du radical menthyle.

Pic à 3,66 p.p.m. attribué à l'hydrogène en position 1 du radical menthyle.

Pics à 6,10-6,11-6,13 p.p.m. attribués à l'hydrogène en position 5 de la furanone.

Pics à 6,16-6,18 ; 6,25-6,26 p.p.m. attribués à l'hydrogène en position 3 de la furanone.

Pics à 7,13-7,15 ; 7,21-7,23 p.p.m. attribués à l'hydrogène en position 4 de la furanone.

Stade B : (1R, 4R, 5S) 6,6-diméthyl 4-[(1R, 2S, 5R) 2-prop-2-yl 5-méthyl cyclohexyloxy] 3-oxa bicyclo [3.1.0] hexan-2-one

a) Préparation du sulfurane

A 500 mg de fluoborate de diphényl isopropyl phosphonium en suspension dans 10 $cm^3$ de tétrahydrofurane, on ajoute à − 80 °C, en une seule fois, 1,2 $cm^3$ de solution, 1,5 M de terbutyl-lithium dans le pentane, agite pendant 30 minutes à − 80 °C.

b) Formation du cyclopropane

On introduit lentement la solution de sulfurane obtenue précédemment, à − 80 °C, dans une solution de 255 mg de 5R-[(1R, 2S, 5R) 2-prop-2-yl 5-méthyl cyclohexyloxy] (5H) furan-2-one (F = 76 °C) dans 10 $cm^3$ de tétrahydrofurane, agite pendant une heure à − 80 °C, verse sur une solution aqueuse de phosphate monosodique, extrait au chlorure de méthylène, lave à l'eau, sèche, concentre à sec, chromatographie sur gel de silice en éluant avec un mélange d'éther de pétrole (eb. = 35-70 °C) et d'éther éthylique (8-2) et obtient 220 mg de (1R, 4R, 5S) 6,6-diméthyl 4-[(1R, 2S, 5R) 2-prop-2-yl 5-méthyl cyclohexyloxy] 3-oxa bicyclo [3.1.0] hexan-2-one ; F = 82 °C. $[\alpha]_D = -195°$ (c = 4 % chloroforme).

Spectre de R.M.N. ($CDCl_3$).

Pics à 0,73-1,0 p.p.m. attribués aux hydrogènes du méthyle en position 6 du radical menthyle et aux hydrogènes des méthyles de l'isopropyle en position 2 du radical menthyle.

Pics à 1,15-1,2 p.p.m. attribués aux hydrogènes des méthyles en position 6 du cycle cyclopropyle.

Pic à 2,0 p.p.m. attribué aux hydrogènes en position 1 et 5 du cycle cyclopropyle.

Pic à 3,53 p.p.m. attribué à l'hydrogène en position 1 du radical menthyle.

Pic à 5,32 p.p.m. attribué à l'hydrogène en position 4 de la copule lactonique.

Exemple 5 : (1R, 4R, 5S) 6,6-diméthyl 4-hydroxy 3-oxa bicyclo [3.1.0] hexan-2-one

Dans une solution de 805 mg de dérivé menthylé (F = 82 °C), obtenu à l'exemple 4, dans 8 $cm^3$ d'acétone, on ajoute lentement 8 $cm^3$ d'une solution aqueuse 0,5 N d'acide chlorhydrique, agite pendant 4 heures à 20 °C, lave à plusieurs reprises à l'éther de pétrole (eb. = 35-70 °C) de manière à éliminer le menthol de la phase aqueuse, concentre la phase aqueuse à sec sous pression réduite et obtient 360 mg de (1R, 4R, 5S) 6,6-diméthyl 4-hydroxy 3-oxa bicyclo [3.1.0] hexan-2-one. F = 116 °C/120 °C.

$[\alpha]_D = -114°$ (c = 1 %, diméthylformamide).

De la phase organique, après élimination des solvants, on récupère le menthol, pratiquement pur avec un rendement quantitatif.

**Revendications** (pour les Etats contractants : BE, CH, DE, GB, IT, LI, NL, SE)

1. Procédé de préparation de composés de configuration (1RS, 4RS, 5SR), (1R, 4R, 5S) ou (1S, 4S, 5R) de formule I :

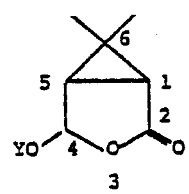

(I)

dans laquelle Y représente un atome d'hydrogène ou le reste organique Z d'un alcool ZOH éventuellement chiral, caractérisé en ce que l'on fait réagir, en présence d'un agent acide, la 5 RS-hydroxy 2,5-dihydrofuran-2-one, de formule II :

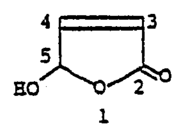

(II)

— soit avec un alcool achiral ZOH, soumet l'éther résultant en milieu anhydre, à l'action d'un isopropylidène sulfurane de formule III :

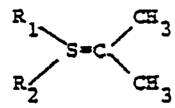

(III)

dans laquelle $R_1$ et $R_2$, identiques ou différents, représentent soit des groupements aromatiques monocycliques éventuellement substitués et notamment des radicaux phényles éventuellement substitués par des méthyles, soit des groupements isopropyles, soit des groupements alcoyles tertiaires, pour obtenir le composé (I) de structure (1RS, 4RS, 5SR) dans lequel Y représente Z, puis hydrolyse, si désiré, ce composé en milieu acide pour obtenir le composé (I) correspondant dans lequel Y représente de l'hydrogène,

— soit avec un alcool chiral optiquement actif ZOH, pour obtenir un mélange d'éthers stéréoisomères dus à l'existence du carbone asymétrique en position 5, mélange qui peut être plus riche en l'un des deux diastéréoisomères, sépare par un moyen physique les éthers diastéréoisomères formés, les fait réagir en milieu anhydre avec un isopropylidène sulfurane de formule (III) pour obtenir les composés de formule I correspondants de configuration (1R, 4R, 5S) ou (1S, 4S, 5R) formule I dans laquelle Y représente Z que l'on hydrolyse, si désiré, en milieu acide pour obtenir les composés (I) correspondants dans lesquels Y représente de l'hydrogène.

2. Procédé selon la revendication 1, caractérisé en ce que l'agent acide en présence duquel on fait réagir la 5RS-hydroxy 2,5-dihydrofuran-2-one et l'alcool ZOH est un acide fort choisi dans le groupe constitué par les acides sulfoniques, l'acide sulfurique, l'acide chlorhydrique et l'acide phosphorique.

3. Procédé selon l'une des revendications 1 ou 2, caractérisé en ce que l'agent acide en présence duquel on fait réagir la 5RS-hydroxy 2,5-dihydrofuran-2-one et l'alcool ZOH est l'acide paratoluène sulfonique.

4. Procédé selon la revendication 1, caractérisé en ce que l'isopropylidène sulfurane est le diphényl isopropylidène sulfurane.

5. Procédé selon la revendication 1, caractérisé en ce que la réaction de l'éther de 5-hydroxy 2,5-dihydrofuran-2-one avec l'isopropylidène sulfurane est effectuée au sein d'un solvant choisi dans le groupe constitué par l'éther diéthylique ou diméthylique du diéthylèneglycol, l'éther éthylique, le diméthylsulfoxyde, le tétrahydrofurane, le diméthoxyéthane.

6. Procédé selon la revendication 1, caractérisé en ce que la réaction de l'éther de 5-hydroxy 2,5-dihydrofuran-2-one avec l'isopropylidène sulfurane est effectuée à une température comprise entre − 90 °C et − 30 °C.

7. Procédé selon la revendication 1, caractérisé en ce que l'hydrolyse des éthers de formule I est effectuée à l'aide d'un acide fort, en présence d'eau et au sein d'un solvant miscible à l'eau et capable de dissoudre le composé à hydrolyser.

8. Procédé selon l'une des revendications 1 ou 7, caractérisé en ce que l'acide fort est choisi dans le groupe constitué par les acides sulfoniques, l'acide sulfurique, l'acide chlorhydrique et l'acide phosphorique.

9. Procédé selon l'une quelconque des revendications 1, 7 ou 8, caractérisé en ce que le solvant au sein duquel est effectuée l'hydrolyse est choisi dans le groupe constitué par les alcanols, le dioxane, le tétrahydrofurane, le diméthylformamide et certaines cétones aliphatiques.

10. Procédé selon la revendication 1, caractérisé en ce que le reste d'alcool achiral Z est un reste méthyle et que l'on fait réagir alors la 5(RS)-méthoxy 2,5-dihydrofuran-2-one avec le diphényl isopropylidène sulfurane, pour obtenir le (1RS, 5SR) 6,6-diméthyl 4-(R, S) méthoxy 3-oxa bicyclo [3.1.0] hexan-2-one, que l'on soumet à une hydrolyse acide pour obtenir le (1RS, 5SR) 6,6-diméthyl 4-(RS) hydroxy 3-oxa bicyclo [3.1.0] hexan-2-one.

11. Procédé selon la revendication 1, caractérisé en ce que le reste d'alcool chiral Z est un reste d'alcool S (α-méthyl 3-phénoxyphényl) méthylique, en ce que l'on fait alors réagir cet alcool avec la 5RS-hydroxy-2,5-dihydrofuran-2-one pour obtenir un mélange de 5R-[1S (3-phénoxyphényl) α-méthyl méthoxy] 2,5-dihydrofuran-2-one et de 5S-[1S (3-phénoxyphényl) α-méthyl méthoxy] 2,5-dihydrofuran-2-one plus riche en isomère « 5R » qu'en isomère « 5 S », en ce que l'on sépare par chromatographie l'isomère 5R-[1S (3-phénoxyphényl) α-méthyl méthoxy] 2,5-dihydrofuran-2-one, en ce que l'on fait réagir ce composé avec le diphényl isopropylidène sulfurane, pour obtenir le (1R, 5S) 6,6-diméthyl 4R-[1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1R, 5S) 6,6-diméthyl 4R-hydroxy 3-oxa bicyclo [3.1.0] hexan-2-one.

12. Procédé selon la revendication 1, caractérisé en ce que l'on opère selon un mode opératoire analogue à celui de la revendication 11, mais en séparant par chromatographie, l'isomère 5S-[1S (3-phénoxyphényl) α-méthyl méthoxy] 2,5-dihydrofuran-2-one pour obtenir, après réaction avec le diphényl isopropylidène sulfurane, le (1S, 5R) 6,6-diméthyl 4S-[1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1S, 5R) 6,6-diméthyl 4(S)-hydroxy 3-oxa bicyclo [3.1.0] hexan-2-one.

13. Procédé selon la revendication 1, caractérisé en ce que le reste d'alcool chiral Z est un reste l-menthyle, en ce que l'on fait réagir cet alcool avec la 5RS-hydroxy 2,5-dihydrofuran-2-one, obtient un

mélange de 5R [1R, 2S, 5R]-2-prop-2-yl 5-méthyl cyclohexyloxy 2,5-dihydrofuran-2-one et de 5S [1R, 2S, 5R]-2-prop-2-yl 5-méthyl cyclohexyloxy 2,5-dihydrofuran-2-one, plus riche en isomère 5R qu'en isomère 5S, en ce que l'on sépare par cristallisation l'isomère 5R [1R, 2S, 5R]-2-prop-2-yl 5-méthyl cyclohexyloxy 2,5-dihydrofuran-2-one, en ce que l'on fait réagir ce composé avec le diphényl isopropylidène sulfurane pour obtenir le (1R, 4R, 5S) 6,6-diméthyl 4-[(1R, 2S, 5R)-2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1R,5S) 6,6-diméthyl 4R-hydroxy 3-oxa bicyclo [3.1.0] hexan-2-one.

14. Procédé selon la revendication 1, caractérisé en ce que l'on opère selon un mode opératoire analogue à celui de la revendication 13, mais après séparation de l'isomère 5R, en isolant par chromatographie le 5S [1R, 2S, 5R]-2-prop-2-yl 5-méthyl cyclohexyloxy 2,5-dihydrofuran-2-one pour obtenir après réaction avec le diphényl isopropylidène sulfurane, le (1S, 4S, 5R) 6,6-diméthyl 4-[(1R, 2S, 5R)-2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1S, 5R) 6,6-diméthyl 4S-hydroxy 3-oxa bicyclo [3.1.0] hexan-2-one.

**Revendications** (pour l'Etat contractant AT)

1. Procédé de préparation de composés de configuration (1RS, 4RS, 5SR), (1R, 4R, 5S) ou (1S, 4S, 5R) de formule I :

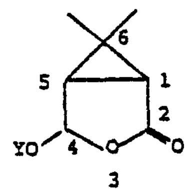

(I)

dans laquelle Y représente un atome d'hydrogène ou le reste organique Z d'un alcool ZOH éventuellement chiral, caractérisé en ce que l'on fait réagir, en présence d'un agent acide, la 5 RS-hydroxy 2,5-dihydrofuran-2-one, de formule II :

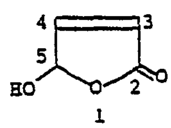

(II)

— soit avec un alcool achiral ZOH, soumet l'éther résultant en milieu anhydre, à l'action d'un isopropylidène sulfurane de formule III :

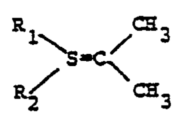

(III)

dans laquelle $R_1$ et $R_2$, identiques ou différents, représentent soit des groupements aromatiques monocycliques éventuellement substitués et notamment des radicaux phényles éventuellement substitués par des méthyles, soit des groupements isopropyles, soit des groupements alcoyles tertiaires, pour obtenir le composé (I) de structure (1RS, 4RS, 5SR) dans lequel Y représente Z, puis hydrolyse, si désiré, ce composé en milieu acide pour obtenir le composé (I) correspondant dans lequel Y représente de l'hydrogène,
— soit avec un alcool chiral optiquement actif ZOH, pour obtenir un mélange d'éthers stéréoisomères dus à l'existence du carbone asymétrique en position 5, mélange qui peut être plus riche en l'un des deux diastéréoisomères, sépare par un moyen physique les éthers diastéréoisomères formés, les fait réagir en milieu anhydre avec un isopropylidène sulfurane de formule (III) pour obtenir les composés de formule I correspondants de configuration (1R, 4R, 5S) ou (1S, 4S, 5R) formule I dans laquelle Y représente Z que l'on hydrolyse, si désiré, en milieu acide pour obtenir les composés (I) correspondants dans lesquels Y représente de l'hydrogène.

2. Procédé selon la revendication 1, caractérisé en ce que l'agent acide en présence duquel on fait réagir la 5RS-hydroxy 2,5-dihydrofuran-2-one et l'alcool ZOH est un acide fort choisi dans le groupe constitué par les acides sulfoniques, l'acide sulfurique, l'acide chlorhydrique et l'acide phosphorique.

3. Procédé selon l'une des revendications 1 ou 2, caractérisé en ce que l'agent acide en présence duquel on fait réagir la 5RS-hydroxy 2,5-dihydrofuran-2-one et l'alcool ZOH est l'acide paratoluène sulfonique.

4. Procédé selon la revendication 1, caractérisé en ce que l'isopropylidène sulfurane est le diphényl isopropylidène sulfurane.

5. Procédé selon la revendication 1, caractérisé en ce que la réaction de l'éther de 5-hydroxy 2,5-dihydrofuran-2-one avec l'isopropylidène sulfurane est effectuée au sein d'un solvant choisi dans le groupe constitué par l'éther diéthylique ou diméthylique du diéthylèneglycol, l'éther éthylique, le diméthylsulfoxyde, le tétrahydrofurane, le diméthoxyéthane.

6. Procédé selon la revendication 1, caractérisé en ce que la réaction de l'éther de 5-hydroxy 2,5-dihydrofuran-2-one avec l'isopropylidène sulfurane est effectuée à une température comprise entre − 90 °C et − 30 °C.

7. Procédé selon la revendication 1, caractérisé en ce que l'hydrolyse des éthers de formule I est effectuée à l'aide d'un acide fort, en présence d'eau et au sein d'un solvant miscible à l'eau et capable de dissoudre le composé à hydrolyser.

8. Procédé selon l'une des revendications 1 ou 7, caractérisé en ce que l'acide fort est choisi dans le groupe constitué par les acides sulfoniques, l'acide sulfurique, l'acide chlorhydrique et l'acide phosphorique.

9. Procédé selon l'une quelconque des revendications 1, 7 ou 8, caractérisé en ce que le solvant au sein duquel est effectuée l'hydrolyse est choisi dans le groupe constitué par les alcanols, le dioxane, le tétrahydrofurane, le diméthylformamide et certaines cétones aliphatiques.

10. Procédé selon la revendication 1, caractérisé en ce que le reste d'alcool achiral Z est un reste méthyle et que l'on fait réagir alors la 5(RS)-méthoxy 2,5-dihydrofuran-2-one avec le diphényl isopropylidène sulfurane, pour obtenir le (1RS, 5SR) 6,6-diméthyl 4-(R, S) méthoxy 3-oxa bicyclo [3.1.0] hexan-2-one, que l'on soumet à une hydrolyse acide pour obtenir le (1RS, 5SR)-6,6-diméthyl 4(RS)-hydroxy 3-oxa bicyclo [3.1.0] hexan-2-one.

11. Procédé selon la revendication 1, caractérisé en ce que le reste d'alcool chiral Z est un reste d'alcool S (α-méthyl 3-phénoxyphényl) méthylique, en ce que l'on fait alors réagir cet alcool avec la 5RS-hydroxy-2,5-dihydrofuran-2-one pour obtenir un mélange de 5R-[1S (3-phénoxyphényl) α-méthyl méthoxy] 2,5-dihydrofuran-2-one et de 5S [1S (3-phénoxyphényl) α-méthyl méthoxy] 2,5-dihydrofuran-2-one plus riche en isomère « 5R » qu'en isomère « 5S », en ce que l'on sépare par chromatographie l'isomère 5R-[1S (3-phénoxyphényl) α-méthyl méthoxy] 2,5-dihydrofuran-2-one, en ce que l'on fait réagir ce composé avec le diphényl isopropylidène sulfurane, pour obtenir le (1R, 5S) 6,6-diméthyl 4R-[1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1R, 5S) 6,6-diméthyl 4R-hydroxy 3-oxa bicyclo [3.1.0] hexan-2-one.

12. Procédé selon la revendication 1, caractérisé en ce que l'on opère selon un mode opératoire analogue à celui de la revendication 11, mais en séparant par chromatographie, l'isomère 5S-[1S (3-phénoxyphényl) α-méthyl méthoxy] 2,5-dihydrofuran-2-one pour obtenir, après réaction avec le diphényl isopropylidène sulfurane, le (1S, 5R) 6,6-diméthyl 4S [1S (3-phénoxyphényl) α-méthyl méthoxy] 3-oxa bicyclo [3.1.0] hexan-2-one et en ce que l'on soumet, si désiré, ce composé à une hydrolyse acide pour obtenir le (1S, 5R) 6,6-diméthyl 4(S)-hydroxy 3-oxa bicyclo [3.1.0] hexan-2-one.

13. Procédé selon la revendication 1, caractérisé en ce que le reste d'alcool chiral Z est un reste l-menthyle, en ce que l'on fait réagir cet alcool avec la 5RS-hydroxy 2,5-dihydrofuran-2-one, obtient un mélange de 5R [1R, 2S, 5R] 2-prop-2-yl 5-méthyl cyclohexyloxy 2,5-dihydrofuran-2-one et de 5S-[1R, 2S, 5R] 2-prop-2-yl 5-méthyl cyclohexyloxy 2,5-dihydrofuran-2-one, plus riche en isomère 5R qu'en isomère 5S, en ce que l'on sépare par cristallisation l'isomère 5R-[1R, 2S, 5R] 2-prop-2-yl 5-méthyl cyclohexyloxy 2,5-dihydrofuran-2-one, en ce que l'on fait réagir ce composé avec le diphényl isopropylidène sulfurane pour obtenir le (1R, 4R, 5S) 6,6-diméthyl 4-[(1R, 2S, 5R) 2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1R, 5S) 6,6-diméthyl 4R-hydroxy 3-oxa bicyclo [3.1.0] hexan-2-one.

14. Procédé selon la revendication 1, caractérisé en ce que l'on opère selon un mode opératoire analogue à celui de la revendication 13, mais après séparation de l'isomère 5R, en isolant par chromatographie le 5S-[1R, 2S, 5R] 2-prop-2-yl 5-méthyl cyclohexyloxy 2,5-dihydrofuran-2-one pour obtenir après réaction avec le diphényl isopropylidène sulfurane, le (1S, 4S, 5R) 6,6-diméthyl 4-[(1R, 2S, 5R) 2-prop-2-yl 5-méthyl cyclohexyloxy] 3-oxa bicyclo [3.1.0] hexan-2-one et en ce que l'on soumet, si désiré, ce composé à une hydrolyse acide pour obtenir le (1S, 5R) 6,6-diméthyl 4S-hydroxy 3-oxa bicyclo [3.1.0] hexan-2-one.

**Claims** (for the contracting states : BE, CH/LI, DE, GB, IT, NL, SE)

1. Process for the preparation of compounds having the configurations (1RS, 4RS, 5SR), (1R, 4R, 5S) or (1S, 4S, 5R) with the formula (I)

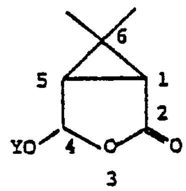

(I)

in which Y represents a hydrogen atom or the organic residue Z of an alcohol ZOH, possibly chiral, characterized in that 5RS-hydroxy 2,5-dihydrofuran-2-one, with the formula (II)

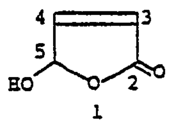

(II)

is made to react in the presence of an acid agent
— either with an achiral alcohol ZOH, the resultant ether being submitted in an anhydrous medium to the action of an isopropylidene sulphurane with the formula (III)

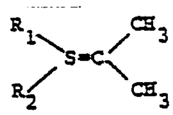

(III)

in which $R_1$ and $R_2$, identical or different, represent either monocyclic aromatic groups, possibly substituted and particularly phenyl radicals possibly substituted by methyls, or isopropyl groups or tertiary alkyl groups, so as to obtain the compound (I) with the structure (1RS, 4RS, 5SR) in which Y represents Z, then, if desired, this compound is hydrolyzed in an acid medium so as to obtain the corresponding compound (I) in which Y represents hydrogen,
— or with an optically active chiral alcohol ZOH, so as to obtain a mixture of stereo-isomeric ethers due to the existence of the asymmetric carbon in position 5, which mixture may be richer in one of the two diastereo-isomers, the diastereo-isomeric ethers formed are separated by a physical means, and made to react in an anhydrous medium with an isopropylidene sulphurane with the formula (III) so as to obtain the corresponding compounds with the formula (I) having the configuration (1R, 4R, 5S) or (1S, 4S, 5R), in which formula (I) Y represents Z, which, if desired, is hydrolyzed in an acid medium so as to obtain the corresponding compounds (I) in which Y represents hydrogen.

2. Process according to claim 1, characterized in that the acid agent in the presence of which the 5RS-hydroxy 2,5-dihydrofuran-2-one and the alcohol ZOR are made to react is a strong acid chosen from the group constituted by sulphonic acids, sulphuric acid, hydrochloric acid and phosphoric acid.

3. Process according to one of the claims 1 or 2, characterized in that the acid agent in the presence of which the 5RS-hydroxy 2,5-dihydrofuran-2-one and the alcohol ZOH are made to react is paratoluene sulphonic acid.

4. Process according to claim 1, characterized in that the isopropylidene sulphurane is diphenyl isopropylidene sulphurane.

5. Process according to claim 1, characterized in that the reaction of the ether of 5-hydroxy 2,5-dihydrofuran-2-one with the isopropylidene sulphurane is carried out in a solvent chosen from the group constituted by the diethyl or dimethyl ether of diethyleneglycol, ethyl ether, dimethylsulphoxide, tetrahydrofuran and dimethoxyethane.

6. Process according to claim 1, characterized in that the reaction of the ether of 5-hydroxy 2,5-dihydrofuran-2-one with the isopropylidene sulphurane is carried at a temperature between − 90 °C and − 30 °C.

7. Process according to claim 1, characterized in that the hydrolysis of the ethers of formula (I) is carried out by a strong acid, in the presence of water and in a solvent miscible with water and capable of dissolving the compound to be hydrolyzed.

8. Process according to one of the claims 1 or 7, characterized in that the strong acid is chosen from the group constituted by sulphonic acids, sulphuric acid, hydrochloric acid and phosphoric acid.

9. Process according to any one of the claims 1, 7 or 8, characterized in that the solvent in which the hydrolysis is carried out is chosen from the group constituted by the alkanols, dioxan, tetrahydrofuran, dimethylformamide and certain aliphatic ketones.

10. Process according to claim 1, characterized in that the achiral alcohol residue Z is a methyl residue and that the 5(RS)-methoxy 2,5-dihydrofuran-2-one is then made to react with diphenyl isopropylidene sulphurane, so as to obtain (1RS, 5SR) 6,6-dimethyl 4-(R,S)-methoxy 3-oxa bicyclo [3.1.0] hexan-2-one, which is submitted to an acid hydrolysis so as to obtain (1RS, 5SR) 6,6-dimethyl 4(RS)-hydroxy 3-oxa bicyclo [3.1.0] hexan-2-one.

11. Process according to claim 1, characterized in that the chiral alcohol residue Z is a residue of S(α-methyl 3-phenoxyphenyl) methyl alcohol, in that this alcohol is then made to react with the 5RS-hydroxy-2,5-dihydrofuran-2-one so as to obtain a mixture of 5R-[1S (3-phenoxyphenyl) α-methyl methoxy] 2,5-dihydrofuran-2-one and of 5S-[1S (3-phenoxyphenyl) α-methyl methoxy] 2,5-dihydrofuran-2-one, richer in isomer « 5R » than in isomer « 5S », in that the isomer 5R-[1S (3-phenoxyphenyl) α-methyl methoxy] 2,5-dihydrofuran-2-one is separated by chromatography, in that this compound is made to react with the diphenyl isopropylidene sulphurane so as to obtain the (1R, 5S) 6,6-dimethyl 4R-[1S (3-phenoxyphenyl) α-methyl methoxy] 3-oxa bicyclo [3.1.0] hexan-2-one and in that, if desired, this compound is submitted to an acid hydrolysis so as to obtain the (1R, 5S) 6,6-dimethyl 4R-hydroxy 3-oxa bicyclo [3.1.0] hexan-2-one.

12. Process according to claim 1, characterized in that the operation is carried out in a similar way to that of claim 11, but separating by chromatography the isomer 5S-[1S (3-phenoxyphenyl) α-methyl methoxy] 2,5-dihydrofuran-2-one so as to obtain, after reaction with the diphenyl isopropylidene sulphurane, the (1S, 5R) 6,6-dimethyl 4S-[1S (3-phenoxyphenyl) α-methyl methoxy] 3-oxa bicyclo [3.1.0] hexan-2-one, and in that, if desired, this compound is submitted to an acid hydrolysis so as to obtain the (1S, 5R) 6,6-dimethyl 4(S)-hydroxy 3-oxa bicyclo [3.1.0] hexan-2-one.

13. Process according to claim 1, characterized in that the chiral alcohol residue Z is a l-menthyl residue, in that this alcohol is made to react with the 5RS-hydroxy 2,5-dihydrofuran-2-one, a mixture of 5R-[1R, 2S, 5R] 2-prop-2-yl 5-methyl cyclohexyloxy 2,5-dihydrofuran-2-one and of 5S-[1R, 2S, 5R] 2-prop-2-yl 5-methyl cyclohexyloxy 2,5-dihydrofuran-2-one is obtained, richer in isomer 5R than in isomer 5S, in that the isomer 5R-[1R, 2S, 5R] 2-prop-2-yl 5-methyl cyclohexyloxy 2,5-dihydrofuran-2-one is separated by crystallization, in that this compound is made to react with the diphenyl isopropylidene sulphurane so as to obtain (1R, 4R, 5S) 6,6-dimethyl 4-[(1R, 2S, 5R) 2-prop-2-yl 5-methyl cyclohexyloxy] 3-oxa bicyclo [3.1.0] hexan-2-one and in that, if desired, this compound is submitted to an acid hydrolysis so as to obtain (1R, 5S) 6,6-dimethyl 4R-hydroxy 3-oxa bicyclo [3.1.0] hexan-2-one.

14. Process according to claim 1, characterized in that the operation is carried out in a similar way to that of claim 13, but after separation of the isomer 5R, the 5S-[1R, 2S, 5R] 2-prop-2-yl 5-methyl cyclohexyloxy 2,5-dihydrofuran-2-one is isolated by chromatography so as to obtain, after reaction with the diphenyl isopropylidene sulphurane (1S, 4S, 5R) 6,6-dimethyl 4-[(1R, 2S, 5R) 2-prop-2-yl 5-methyl cyclohexyloxy] 3-oxa bicyclo [3.1.0] hexan-2-one, and in that, if desired, this compound is submitted to an acid hydrolysis so as to obtain (1S, 5R) 6,6-dimethyl 4S-hydroxy 3-oxa bicyclo [3.1.0] hexan-2-one.

**Claims** (for the contracting state AT)

1. Process for the preparation of compounds having the configurations (1RS, 4RS, 5SR), (1R, 4R, 5S) or (1S, 4S, 5R) with the formula (I)

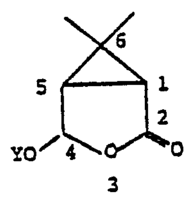

(I)

in which Y represents a hydrogen atom or the organic residue Z of an alcohol ZOH, possibly chiral, characterized in that 5RS-hydroxy 2,5-dihydrofuran-2-one, with the formula (II)

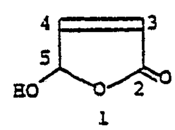

(II)

is made to react in the presence of an acid agent
— either with an achiral alcohol ZOH, the resultant ether being submitted in an anhydrous medium to the action of an isopropylidene sulphurane with the formula (III)

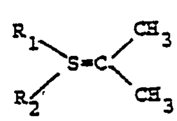

(III)

in which $R_1$ and $R_2$, identical or different, represent either monocyclic aromatic groups, possibly substituted and particularly phenyl radicals possibly substituted by methyls, or isopropyl groups or tertiary alkyl groups, so as to obtain the compound (I) with the structure (1RS, 4RS, 5SR) in which Y represents Z, then, if desired, this compound is hydrolyzed in an acid medium so as to obtain the

corresponding compound (I) in which Y represents hydrogen,

— or with an optically active chiral alcohol ZOH, so as to obtain a mixture of stereo-isomeric ethers due to the existence of the asymmetric carbon in position 5, which mixture may be richer in one of the two diastereo-isomers, the diastereo-isomeric ethers formed are separated by a physical means, and made to react in an anhydrous medium with an isopropylidene sulphurane with the formula (III) so as to obtain the corresponding compounds with the formula (I) having the configuration (1R, 4R, 5S) or (1S, 4S, 5R), in which formula (I) Y represents Z, which, if desired, is hydrolyzed in an acid medium so as to obtain the corresponding compounds (I) in which Y represents hydrogen.

2. Process according to claim 1, characterized in that the acid agent in the presence of which the 5RS-hydroxy 2,5-dihydrofuran-2-one and the alcohol ZOR are made to react is a strong acid chosen from the group constituted by sulphonic acids, sulphuric acid, hydrochloric acid and phosphoric acid.

3. Process according to one of the claims 1 or 2, characterized in that the acid agent in the presence of which the 5RS-hydroxy 2,5-dihydrofuran-2-one and the alcohol ZOH are made to react is paratoluene sulphonic acid.

4. Process according to claim 1, characterized in that the isopropylidene sulphurane is diphenyl isopropylidene sulphurane.

5. Process according to claim 1, characterized in that the reaction of the ether of 5-hydroxy 2,5-dihydrofuran-2-one with the isopropylidene sulphurane is carried out in a solvent chosen from the group constituted by the diethyl or dimethyl ether of diethyleneglycol, ethyl ether, dimethylsulphoxide, tetrahydrofuran and dimethoxyethane.

6. Process according to claim 1, characterized in that the reaction of the ether 5-hydroxy 2,5-dihydrofuran-2-one with the isopropylidene sulphurane is carried at a temperature between − 90 °C and − 30 °C.

7. Process according to claim 1, characterized in that the hydrolysis of the ethers of formula (I) is carried out by a strong acid, in the presence of water and in a solvent miscible with water and capable of dissolving the compound to be hydrolyzed.

8. Process according to one of the claims 1 or 7, characterized in that the strong acid is chosen from the group constituted by sulphonic acids, sulphuric acid, hydrochloric acid and phosphoric acid.

9. Process according to any one of the claims 1, 7 or 8, characterized in that the solvent in which the hydrolysis is carried out is chosen from the group constituted by the alkanols, dioxan, tetrahydrofuran, dimethylformamide and certain aliphatic ketones.

10. Process according to claim 1, characterized in that the achiral alcohol residue Z is a methyl residue and that the 5(RS)-methoxy 2,5-dihydrofuran-2-one is then made to react with diphenyl isopropylidene sulphurane, so as to obtain (1RS, 5SR) 6,6-dimethyl 4-(R, S)-methoxy 3-oxa bicyclo [3.1.0] hexan-2-one, which is submitted to an acid hydrolysis so as to obtain (1RS, 5SR) 6,6-dimethyl 4(RS)-hydroxy 3-oxa bicyclo [3.1.0] hexan-2-one.

11. Process according to claim 1, characterized in that the chiral alcohol residue Z is a residue of S (α-methyl 3-phenoxyphenyl) methyl alcohol, in that this alcohol is then made to react with the 5RS-hydroxy-2,5-dihydrofuran-2-one so as to obtain a mixture of 5R-[1S (3-phenoxyphenyl) α-methyl methoxy] 2,5-dihydrofuran-2-one and of 5S-[1S (3-phenoxyphenyl) α-methyl methoxy] 2,5-dihydrofuran-2-one, richer in isomer « 5R » than in isomer « 5S », in that the isomer 5R-[1S (3-phenoxyphenyl) α-methyl methoxy] 2,5-dihydrofuran-2-one is separated by chromatography, in that this compound is made to react with the diphenyl isopropylidene sulphurane so as to obtain the (1R, 5S) 6,6-dimethyl 4R-[1S (3-phenoxyphenyl) α-methyl methoxy] 3-oxa bicyclo [3.1.0] hexan-2-one and in that, if desired, this compound is submitted to an acid hydrolysis so as to obtain the (1R, 5S) 6,6-dimethyl 4R-hydroxy 3-oxa bicyclo [3.1.0] hexan-2-one.

12. Process according to claim 1, characterized in that the operation is carried out in a similar way to that of claim 11, but separating by chromatography the isomer 5S-[1S (3-phenoxyphenyl) α-methyl methoxy] 2,5-dihydrofuran-2-one so as to obtain, after reaction with the diphenyl isopropylidene sulphurane, the (1S, 5R) 6,6-dimethyl 4S-[(1S (3-phenoxyphenyl) α-methyl methoxy] 3-oxa bicyclo [3.1.0] hexan-2-one, and in that, if desired, this compound is submitted to an acid hydrolysis so as to obtain the (1S, 5R) 6,6-dimethyl 4(S)-hydroxy 3-oxa bicyclo [3.1.0] hexan-2-one.

13. Process according to claim 1, characterized in that the chiral alcohol residue Z is a l-menthyl residue, in that this alcohol is made to react with the 5RS-hydroxy 2,5-dihydrofuran-2-one, a mixture of 5R-[1R, 2S, 5R] 2-prop-2-yl 5-methyl cyclohexyloxy 2,5-dihydrofuran-2-one and of 5S-[1R, 2S, 5R] 2-prop-2-yl 5-methyl cyclohexyloxy 2,5-dihydrofuran-2-one is obtained, richer in isomer 5R than in isomer 5S, in that the isomer 5R-[1R, 2S, 5R] 2-prop-2-yl 5-methyl cyclohexyloxy 2,5-dihydrofuran-2-one is separated by crystallization, in that this compound is made to react with the diphenyl isopropylidene sulphurane so as to obtain (1R, 4R, 5S) 6,6-dimethyl 4-[(1R, 2S, 5R) 2-prop-2-yl 5-methyl cyclohexyloxy] 3-oxa bicyclo [3.1.0] hexan-2-one and in that, if desired, this compound is submitted to an acid hydrolysis so as to obtain (1R, 5S) 6,6-dimethyl 4R-hydroxy 3-oxa bicyclo [3.1.0] hexan-2-one.

14. Process according to claim 1, characterized in that the operation is carried out in a similar way to that of claim 13, but after separation of the isomer 5R, the 5S-[1R, 2S, 5R] 2-prop-2-yl 5-methyl cyclohexyloxy 2,5-dihydrofuran-2-one is isolated by chromatography so as to obtain, after reaction with the diphenyl isopropylidene sulphurane, (1S, 4S, 5R) 6,6-dimethyl 4-[(1R, 2S, 5R) 2-prop-2-yl 5-methyl

cyclohexyloxy] 3-oxa bicyclo [3.1.0] hexan-2-one, and in that, if desired, this compound is submitted to an acid hydrolysis so as to obtain (1S, 5R) 6,6-dimethyl 4S-hydroxy 3-oxa bicyclo [3.1.0] hexan-2-one.

**Ansprüche** (für die Vertragsstaaten : BE, CH/LI, DE, GB, IT, NL, SE)

1. Verfahren zur Herstellung von Verbindungen mit (1RS, 4RS, 5SR)-, (1R, 4R, 5S)- oder (1S, 4S, 5R)-Konfiguration der Formel I

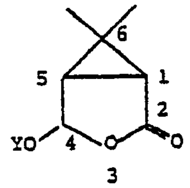

(I)

worin Y ein Wasserstoffatom oder den organischen Rest Z eines gegebenenfalls chiralen Alkohols ZOH bedeutet, dadurch gekennzeichnet, daß man in Gegenwart eines sauren Mittels 5RS-Hydroxy-2,5-dihydrofuran-2-on der Formel II

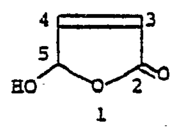

(II)

entweder mit einem achiralen Alkohol ZOH umsetzt, den erhaltenen Äther in wasserfreiem Milieu der Einwirkung eines Isopropylidensulfurans der Formel III

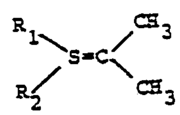

(III)

unterzieht, worin $R_1$ und $R_2$, die gleich oder verschieden sein können, entweder gegebenenfalls substituierte monocyclische aromatische Gruppen und insbesondere gegebenenfalls durch Methylreste substituierte Phenylreste oder Isopropylgruppen oder tertiäre Alkylgruppen bedeuten, um die Verbindung I mit (1RS, 4RS, 5SR)-Struktur, worin Y Z bedeutet, zu erhalten und danach gewünschtenfalls diese Verbindung in saurem Milieu hydrolysiert, um die entsprechende Verbindung I zu erhalten, worin Y Wasserstoff bedeutet,
oder mit einem optisch aktiven chiralen Alkohol ZOH umsetzt, um ein Gemisch der stereoisomeren Äther aufgrund des Vorliegens eines asymmetrischen Kohlenstoffatoms in 5-Stellung zu erhalten, welches Gemisch an einem der beiden Diastereomeren reicher sein kann, mit Hilfe einer physikalischen Methode die gebildeten diastereomeren Äther trennt, sie in wasserfreiem Milieu mit einem Isopropylidensulfuran der Formel III umsetzt, um die entsprechenden Verbindungen mit (1R, 4R, 5S)- oder (1S, 4S, 5R)-Konfiguration der Formel I, worin Y Z bedeutet, zu erhalten, welche man gewünschtenfalls in saurem Milieu hydrolysiert, um die entsprechenden Verbindungen I zu erhalten, worin Y Wasserstoff bedeutet.

2. Verfahren gemäß Anspruch 1, dadurch gekennzeichnet, daß das saure Mittel, in dessen Gegenwart man 5RS-Hydroxy-2,5-dihydrofuran-2-on und den Alkohol ZOH umsetzt, eine starke Säure ist ausgewählt unter den Sulfonsäuren, der Schwefelsäure, der Chlorwasserstoffsäure und der Phosphorsäure.

3. Verfahren gemäß einem der Ansprüche 1 oder 2, dadurch gekennzeichnet, daß das saure Mittel, in dessen Gegenwart man 5RS-Hydroxy-2,5-dihydrofuran-2-on und den Alkohol ZOH umsetzt p-Toluolsulfonsäure ist.

4. Verfahren gemäß Anspruch 1, dadurch gekennzeichnet, daß das Isopropylidensulfuran Diphenylisopropylidensulfuran ist.

5. Verfahren gemäß Anspruch 1, dadurch gekennzeichnet, daß die Umsetzung des Äthers des 5-Hydroxy-2,5-dihydrofuran-2-ons mit dem Isopropylidensulfuran in dem Medium eines Lösungsmittels durchgeführt wird, ausgewählt unter dem Diäthylenglykoldiäthyl- oder -dimethyläther, dem Äthyläther, Dimethylsulfoxid, Tetrahydrofuran und Dimethoxyäthan.

6. Verfahren gemäß Anspruch 1, dadurch gekennzeichnet, daß die Umsetzung des Äthers des 5-Hydroxy-2,5-dihydrofuran-2-ons mit dem Isopropylidensulfuran bei einer Temperatur zwischen – 90 °C und – 30 °C durchgeführt wird.

7. Verfahren gemäß Anspruch 1, dadurch gekennzeichnet, daß die mittels, das mit Wasser mischbar ist und befähigt ist, die zu hydrolysierende Verbindung aufzulösen, durchgeführt wird.

8. Verfahren gemäß einem der Ansprüche 1 oder 7, dadurch gekennzeichnet, daß die starke Säure ausgewählt wird unter den Sulfonsäuren, der Schwefelsäure, der Chlorwasserstoffsäure und der Phosphorsäure.

9. Verfahren gemäß einem der Ansprüche 1, 7 oder 8, dadurch gekennzeichnet, daß das Lösungsmittel, in dessen Medium man die Hydrolyse durchführt, ausgewählt wird unter den Alkanolen, dem Dioxan, dem Tetrahydrofuran, dem Dimethylformamid und gewissen aliphatischen Ketonen.

10. Verfahren gemäß Anspruch 1, dadurch gekennzeichnet, daß der Rest des achiralen Alkohols Z ein Methylrest ist und daß man dann 5(RS)-Methoxy-2,5-dihydrofuran-2-on mit Diphenylisopropylidensulfuran umsetzt, um (1RS, 5SR) 6,6-Dimethyl-4-(RS)-methoxy-3-oxabicyclo-[3.1.0]-hexan-2-on zu erhalten, das man einer sauren Hydrolyse unterzieht, um (1RS, 5SR) 6,6-Dimethyl-4-(RS)-hydroxy-3-oxabicyclo-[3.1.0]-hexan-2-on zu erhalten.

11. Verfahren gemäß Anspruch 1, dadurch gekennzeichnet, daß der Rest des chiralen Alkohols Z ein Rest des S-(α-Methyl-3-phenoxyphenyl)-methylalkohols ist, daß man dann diesen Alkohol mit 5RS-Hydroxy-2,5-dihydrofuran-2-on umsetzt, um ein Gemisch von 5R-[1S-(3-Phenoxyphenyl)-α-methylmethoxy]-2,5-dihydrofuran-2-on und 5S-[1S-(3-Phenoxyphenyl)-α-methylmethoxy]-2,5-dihydrofuran-2-on zu erhalten, das reicher ist an dem « 5R »-Isomeren als an dem « 5S »-Isomeren, daß man durch Chromatographie das 5R-[1S-(3-Phenoxyphenyl)-α-methylmethoxy]-2,5-dihydrofuran-2-on-Isomere abtrennt, daß man diese Verbindung mit Diphenylisopropylidensulfuran umsetzt, um das (1R, 5S) 6,6-Dimethyl-4R-[1S-(3-phenoxyphenyl)-α-methylmethoxy]-3-oxabicyclo-[3.1.0]-hexan-2-on zu erhalten und daß man gewünschtenfalls diese Verbindung einer sauren Hydrolyse unterzieht, um (1R, 5S) 6,6-Dimethyl-4R-hydroxy-3-oxabicyclo-[3.1.0]-hexan-2-on zu erhalten.

12. Verfahren gemäß Anspruch 1, dadurch gekennzeichnet, daß man gemäß einer Arbeitsmethode analog derjenigen von Anspruch 11 arbeitet, wobei man jedoch durch Chromatographie das 5S-[1S-(3-Phenoxyphenyl)-α-methylmethoxy]-2,5-dihydrofuran-2-on-Isomere abtrennt, um nach Umsetzung mit Diphenylisopropylidensulfuran (1S, 5R) 6,6-Dimethyl-4S-[1S-(3-phenoxyphenyl)-α-methylmethoxy]-3-oxabicyclo-[3.1.0]-hexan-2-on zu erhalten und daß man gewünschtenfalls diese Verbindung einer sauren Hydrolyse unterzieht, um (1S, 5R) 6,6-Dimethyl-4(S)-hydroxy-3-oxabicyclo-[3.1.0]-hexan-2-on zu erhalten.

13. Verfahren gemäß Anspruch 1, dadurch gekennzeichnet, daß der Rest des chiralen Alkohols Z ein l-Menthylrest ist, daß man diesen Alkohol mit 5RS-Hydroxy-2,5-dihydrofuran-2-on umsetzt, ein Gemisch von 5R-[1R, 2S, 5R]-2-Prop-2-yl-5-methylcyclohexyloxy-2,5-dihydrofuran-2-on und 5S-[1R, 2S, 5R]-2-Prop-2-yl-5-methylcyclohexyloxy-2,5-dihydrofuran-2-on erhält, das reicher ist an dem 5R-Isomeren als an dem 5S-Isomeren, daß man durch Kristallisation das 5R-[1R, 2S, 5R]-2-Prop-2-yl-5-methylcyclohexyloxy-2,5-dihydrofuran-2-on-Isomere abtrennt, daß man diese Verbindung mit Diphenylisopropylidensulfuran umsetzt, um (1R, 4R, 5S) 6,6-Dimethyl-4-[(1R, 2S, 5R)-2-prop-2-yl-5-methylcyclohexyloxy]-3-oxabicyclo-[3.1.0]-hexan-2-on zu erhalten, daß man gewünschtenfalls diese Verbindung einer sauren Hydrolyse unterzieht, um (1R, 5S) 6,6-Dimethyl-4R-hydroxy-3-oxabicyclo-[3.1.0]-hexan-2-on zu erhalten.

14. Verfahren gemäß Anspruch 1, dadurch gekennzeichnet, daß man gemäß einer Arbeitsmethode analog derjenigen von Anspruch 13 arbeitet, wobei jedoch Abtrennung des 5R-Isomeren man durch Chromatographie das 5S-[1R, 2S, 5R]-2-Prop-2-yl-5-methylcyclohexyloxy-2,5-dihydrofuran-2-on isoliert, um nach Umsetzung mit Diphenylisopropylidensulfuran (1S, 4S, 5R) 6,6-Dimethyl-4-[(1R, 2S, 5R)-2-prop-2-yl-5-methylcyclohexyloxy]-3-oxabicyclo-[3.1.0]-hexan-2-on zu erhalten und man gewünschtenfalls diese Verbindung einer sauren Hydrolyse unterzieht, um (1S, 5R) 6,6-Dimethyl-4S-hydroxy-3-oxabicyclo-[3.1.0]-hexan-2-on zu erhalten.

**Ansprüche** (für den Vertragsstaat AT)

1. Verfahren zur Herstellung von Verbindungen mit (1RS, 4RS, 5SR)-, (1R, 4R, 5S)- oder (1S, 4S, 5R)-Konfiguration der Formel I

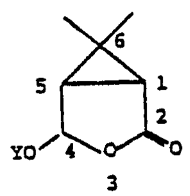

(I)

worin Y ein Wasserstoffatom oder den organischen Rest Z eines gegebenenfalls chiralen Alkohols ZOH bedeutet, dadurch gekennzeichnet, daß man in Gegenwart eines sauren Mittels 5RS-Hydroxy-2,5-dihydrofuran-2-on der Formel II

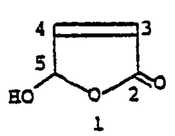

(II)

entweder mit einem achiralen Alkohol ZOH umsetzt, den erhaltenen Äther in wasserfreiem Milieu der Einwirkung eines Isopropylidensulfurans der Formel III

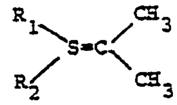

(III)

unterzieht, worin $R_1$ und $R_2$, die gleich oder verschieden sein können, entweder gegebenenfalls substituierte monocyclische aromatische Gruppen und insbesondere gegebenenfalls durch Methylgruppen substituierte Phenylreste oder Isopropylgruppen oder tertiäre Alkylgruppen bedeuten, um die Verbindung I mit (1RS, 4RS, 5SR)-Struktur zu erhalten, worin Y Z bedeutet und danach gewünschtenfalls diese Verbindung in saurem Milieu hydrolysiert, um die entsprechende Verbindung I zu erhalten, worin Y Wasserstoff bedeutet,

oder mit einem optisch aktiven chiralen Alkohol ZOH umsetzt, um ein Gemisch der stereoisomeren Äther aufgrund des Vorliegens eines asymmetrischen Kohlenstoffatoms in 5-Stellung zu erhalten, welches Gemisch an einem der beiden Diastereomeren reicher sein kann, mit Hilfe einer physikalischen Methode die gebildeten diastereomeren Äther trennt, sie in wasserfreiem Milieu mit einem Isopropylidensulfuran der Formel III umsetzt, um die entsprechenden Verbindungen mit (1R, 4R, 5S)- oder (1S, 4S, 5R)-Konfiguration der Formel I, worin Y Z bedeutet, zu erhalten, welche man gewünschtenfalls in saurem Milieu hydrolysiert, um die entsprechenden Verbindungen I zu erhalten, worin Y Wasserstoff bedeutet.

2. Verfahren gemäß Anspruch 1, dadurch gekennzeichnet, daß das saure Mittel, in dessen Gegenwart man 5RS-Hydroxy-2,5-dihydrofuran-2-on und den Alkohol ZOH umsetzt, eine starke Säure ist ausgewählt unter den Sulfonsäuren, der Schwefelsäure, der Chlorwasserstoffsäure und der Phosphorsäure.

3. Verfahren gemäß einem der Ansprüche 1 oder 2, dadurch gekennzeichnet, daß das saure Mittel, in dessen Gegenwart man 5RS-Hydroxy-2,5-dihydrofuran-2-on und den Alkohol ZOH umsetzt p-Toluolsulfonsäure ist.

4. Verfahren gemäß Anspruch 1, dadurch gekennzeichnet, daß das Isopropylidensulfuran Diphenylisopropylidensulfuran ist.

5. Verfahren gemäß Anspruch 1, dadurch gekennzeichnet, daß die Umsetzung des Äthers des 5-Hydroxy-2,5-dihydrofuran-2-ons mit dem Isopropylidensulfuran in dem Medium eines Lösungsmittels durchgeführt wird, ausgewählt unter dem Diäthylenglykoldiäthyl- oder -dimethyläther, dem Äthyläther, Dimethylsulfoxid, Tetrahydrofuran und Dimethoxyäthan.

6. Verfahren gemäß Anspruch 1, dadurch gekennzeichnet, daß die Umsetzung des Äthers des 5-Hydroxy-2,5-dihydrofuran-2-ons mit dem Isopropylidensulfuran bei einer Temperatur zwischen $-90$ °C und $-30$ °C durchgeführt wird.

7. Verfahren gemäß Anspruch 1, dadurch gekennzeichnet, daß die mittels, das mit Wasser mischbar ist und befähigt ist, die zu hydrolysierende Verbindung aufzulösen, durchgeführt wird.

8. Verfahren gemäß einem der Ansprüche 1 oder 7, dadurch gekennzeichnet, daß die starke Säure ausgewählt wird unter den Sulfonsäuren, der Schwefelsäure, der Chlorwasserstoffsäure und der Phosphorsäure.

9. Verfahren gemäß einem der Ansprüche 1, 7 oder 8, dadurch gekennzeichnet, daß das Lösungsmittel, in dessen Medium man die Hydrolyse durchführt, ausgewählt wird unter den Alkanolen, dem Dioxan, dem Tetrahydrofuran, dem Dimethylformamid und gewissen aliphatischen Ketonen.

10. Verfahren gemäß Anspruch 1, dadurch gekennzeichnet, daß der Rest des achiralen Alkohols Z ein Methylrest ist und daß man dann 5(RS)-Methoxy-2,5-dihydrofuran-2-on mit Diphenylisopropylidensulfuran umsetzt, um (1RS, 5SR) 6,6-Dimethyl-4-(RS)-methoxy-3-oxabicyclo-[3.1.0]-hexan-2-on zu erhalten, das man einer sauren Hydrolyse unterzieht, um (1RS, 5SR) 6,6-Dimethyl-4(RS)-hydroxy-3-oxabicyclo-[3.1.0]-hexan-2-on zu erhalten.

11. Verfahren gemäß Anspruch 1, dadurch gekennzeichnet, daß der Rest des chiralen Alkohols Z ein Rest des S-(α-Methyl-3-phenoxyphenyl)-methylalkohols ist, daß man dann diesen Alkohol mit 5RS-Hydroxy-2,5-dihydrofuran-2-on umsetzt, um ein Gemisch von 5R-[1S-(3-Phenoxyphenyl)-α-methylmethoxy]-2,5-dihydrofuran-2-on und 5S-[1S-(3-Phenoxyphenyl)-α-methyl-methoxy]-2,5-dihydrofuran-2-on zu erhalten, das reicher ist an dem « 5R »-Isomeren als an dem « 5S »-Isomeren, daß man durch Chromatographie das 5R-[1S-(3-Phenoxyphenyl)-α-methylmethoxy]-2,5-dihydrofuran-2-on-Isomere abtrennt, daß man diese Verbindung mit Diphenylisopropylidensulfuran umsetzt, um das (1R, 5S) 6,6-Dimethyl-4R-[1S-(3-phenoxyphenyl)-α-methylmethoxy]-3-oxabicyclo-[3.1.0]-hexan-2-on zu erhalten und daß man gewünschtenfalls diese Verbindung einer sauren Hydrolyse unterzieht, um (1R, 5S) 6,6-Dimethyl-4R-hydroxy-(1R, 5S) 6,6-Dimethyl-4R-[1S-(3-phenoxyphenyl)-α-methylmethoxy]-3-oxabicyclo-[3.1.0]-hexan-2-on zu erhalten und daß man gewünschtenfalls diese Verbindung einer sauren Hydrolyse unterzieht, um (1R, 5S) 6,6-Dimethyl-4R-hydroxy-3-oxabicyclo-[3.1.0]-hexan-2-on zu erhalten.

12. Verfahren gemäß Anspruch 1, dadurch gekennzeichnet, daß man gemäß einer Arbeitsweise analog derjenigen von Anspruch 11 arbeitet, wobei man jedoch durch Chromatographie das 5S-[1S-(3-Phenoxyphenyl)-α-methylmethoxy]-2,5-dihydrofuran-2-on-Isomere abtrennt, um nach Umsetzung mit

Diphenylisopropylidensulfuran (1S, 5R) 6,6-Dimethyl-4S-[1S-(3-phenoxyphenyl)-α-methylmethoxy]-3-oxabicyclo-[3.1.0]-hexan-2-on zu erhalten und man gewünschtenfalls diese Verbindung einer sauren Hydrolyse unterzieht, um (1S, 5R) 6,6-Dimethyl-4(S)-hydroxy-3-oxabicyclo-[3.1.0]-hexan-2-on zu erhalten.

13. Verfahren gemäß Anspruch 1, dadurch gekennzeichnet, daß der Rest des chiralen Alkohols Z ein l-Menthylrest ist und daß man diesen Alkohol mit 5RS-Hydroxy-2,5-dihydrofuran-2-on umsetzt, ein Gemisch von 5R-[1R, 2S, 5R]-2-Prop-2-yl-5-methylcyclohexyloxy-2,5-dihydrofuran-2-on und 5S-[1R,2S, 5R]-2-Prop-2-yl-5-methylcyclohexyloxy-2,5-dihydrofuran-2-on erhält, das reicher ist an dem 5R-Isomeren als an dem 5S-Isomeren, daß man durch Kristallisation das 5R-[1R, 2S, 5R]-2-Prop-2-yl-5-methylcyclohexyloxy-2,5-dihydrofuran-2-on abtrennt, daß man diese Verbindung mit Diphenylisopropylidensulfuran umsetzt, um (1R, 4R, 5S) 6,6-Dimethyl-4-[(1R, 2S, 5R)-2-prop-2-yl-5-methylcyclohexyloxy]-3-oxabicyclo-[3.1.0]-hexan-2-on zu erhalten und daß man gewünschtenfalls diese Verbindung einer sauren Hydrolyse unterzieht, um (1R, 5S) 6,6-Dimethyl-4R-hydroxy-3-oxabicyclo-[3.1.0]-hexan-2-on zu erhalten.

14. Verfahren gemäß Anspruch 1, dadurch gekennzeichnet, daß man gemäß einer Arbeitsweise analog derjenigen von Anspruch 13 arbeitet, wobei jedoch nach Abtrennen des 5R-Isomeren man durch Chromatographie das 5S-[1R, 2S, 5R)-2-Prop-2-yl-5-methylcyclohexyloxy]-2,5-dihydrofuran-2-on isoliert, um nach Umsetzung mit Diphenylisopropylidensulfuran (1S, 4S, 5R) 6,6-Dimethyl-4-[(1R, 2S, 5R)-2-prop-2-yl-5-methylcyclohexyloxy]-3-oxabicyclo-[3.1.0]-hexan-2-on zu erhalten und daß man gewünschtenfalls diese Verbindung einer sauren Hydrolyse unterzieht, um (1S, 5R) 6,6-Dimethyl-4S-hydroxy-3-oxabicyclo-[3.1.0]-hexan-2-on zu erhalten.